# DEMANDE DE BREVET EUROPEEN

(11) **EP 4 338 670 A1**
(43) Date de publication de la demande: **20.03.2024**
(21) Numéro de dépôt: 23197201.9
(22) Date de dépôt: 13.09.2023
(51) Int. Cl.: A61B 5/262, A61B 5/268, A61B 5/00

(54) **DISPOSITIF DE TYPE ÉLECTRODE, SON PROCÉDÉ DE PRÉPARATION ET SES UTILISATIONS**

(30) Priorité: 14.09.2022 FR 2209256
(71) Demandeur: Commissariat à l'énergie atomique et aux énergies alternatives, 75015 Paris (FR)
(72) Inventeur: JUILLARD, Sacha, 38054 GRENOBLE CEDEX 09 (FR); TEXIER-NOGUES, Isabelle, 38054 GRENOBLE CEDEX 09 (FR); PLANAT-CHRETIEN, Anne, 38054 GRENOBLE CEDEX 09 (FR)
(74) Mandataire: Brevalex

(57) **Abrégé**

La présente invention concerne un dispositif du type électrode comprenant i) un patch ayant une première et une deuxième faces opposées, ladite première face présentant des nano-structures et/ou des micro-structures, ledit patch, lesdites nano-structures et lesdites micro-structures étant en un matériau comprenant au moins un polymère réticulé, et ii) un élément de reprise de contact électrique, appliqué sur ou intégré au niveau de ladite deuxième face. La présente invention concerne également un procédé de préparation d'un tel dispositif et ses utilisations.

## Description

### DOMAINE TECHNIQUE

La présente invention concerne le domaine des dispositifs médicaux et notamment le domaine des électrodes destinées à des mesures électriques *on vivo* telles que, par exemple, les ElectroEncéphaloGrammes (EEG), les ElectroCardioGrammes (ECG), les ElectroMyoGrammes (EMG) et les mesures de bio-impédance.

Plus particulièrement, l'invention consiste en un dispositif sous forme de patch positionnable directement sur la peau du patient à l'instar d'une électrode classique, sans manipulation supplémentaire. Ce dispositif présente, au niveau de la surface en contact avec la peau du patient, des nano-structures et/ou des micro-structures aptes à percer le *stratum corneum* et à mettre en contact le liquide interstitiel présent au niveau des couches sous-cutanées de la peau avec le polymère réticulé, non-conducteur de l'électricité et biodégradable, qui est l'un des constituants du patch. Suite à cette mise en contact, le polymère réticulé non-conducteur se gonfle de liquide interstitiel et le patch devient ainsi conducteur.

La présente invention concerne également le procédé de préparation d'un tel dispositif et ses utilisations.

### ÉTAT DE LA TECHNIQUE ANTÉRIEURE

En ce qui concerne les électrodes connues dans l'état de la technique, on trouve les électrodes extra-dermiques et les électrodes intra-dermiques en matériaux conducteurs ou semi-conducteurs.

Dans une mesure électrique classique sur patient, les électrodes extra-dermiques sont placées sur la couche externe de la peau i.e. le *stratum corneum.* Cette couche composée de cellules mortes non-hydratées apporte une forte impédance électrique et donc une dégradation du rapport signal sur bruit ainsi qu'une variabilité de la qualité de mesure en fonction des sites de mesures et des individus. Afin de s'affranchir de l'impédance du *stratum corneum,* il est possible de pratiquer l'ablation de ce dernier, le plus souvent par abrasion, ce qui est gourmand en temps et représente un risque infectieux pour le patient.

De plus les électrodes extra-dermiques humides [1] qui représentent la majorité des électrodes commerciales, ont un problème de séchage diminuant la qualité de mesure dans le temps tandis que les électrodes extra-dermiques sèches [2] sont particulièrement sensibles au placement de celles-ci et au mouvement du patient.

Afin de s'affranchir du *stratum corneum* impédant, il est possible d'employer des électrodes intradermiques en matériaux conducteurs sous forme de micro-aiguilles capables de perforer celui-ci **[3].** Ces électrodes peuvent être composées de métal ou d'un matériau support, le plus souvent en silicium ou en polymère, ensuite métallisé. Ces électrodes permettent une très bonne qualité de mesure mais leur rigidité apporte un risque de casse, en particulier en cas de mouvement. Ce risque de casse est d'autant plus problématique que ces matériaux ne se dégradent pas en conditions physiologiques et certains ne sont pas considérés comme biocompatibles par la FDA. Ce type d'électrodes est également le plus souvent associé à des procédés de fabrication sous vide et de micro-usinage relativement coûteux.

Par ailleurs, on connaît des micro-aiguilles d'hydrogels à gonflement, employées en grande majorité dans un contexte de délivrance de principe actif et/ou d'extraction de liquide interstitiel **[4].** Leur principe de fonctionnement repose sur le fait que les aiguilles sèches sont suffisamment rigides pour percer le *stratum corneum* et vont par la suite se gonfler de liquide interstitiel et ainsi permettre leur fonction de délivrance ou d'extraction de ce dernier.

Enfin, des micro-aiguilles poreuses ont été utilisées pour l'iontophorèse par électro-osmose. Un courant continu a été appliqué aux bornes d'un patch micro-aiguilles afin de faciliter la délivrance de principes actifs ou l'extraction de fluides interstitiels **[5].** Ce patch est réalisé à partir de matériaux polymères biodégradables sous formes d'aiguilles poreuses en mélangeant du poly(acide lactique-*co*-acide glycolique) (PGLA) et du 1,4-dioxane puis en congelant l'ensemble avant de sublimer les particules de 1,4-dioxane. Ainsi, la mise en oeuvre de ces micro-aiguilles poreuses est relativement complexe à cause de la nécessité d'introduire une porosité durant le moulage. La résistance mécanique des micro-aiguilles en PGLA est renforcée en revêtant ce polymère avec de la carboxyméthylcellulose (CMC), cette dernière étant déposée sous forme d'un revêtement à l'aide d'un surfactant. De plus, avant application, ces patches doivent être imbibés d'une solution saline et être connectés par des ponts ioniques en agar afin de diminuer la résistance de contact. Cette préparation est relativement laborieuse.

Par conséquent, la mesure électrique classique i.e. à l'aide d'électrodes extra-dermiques est dégradée par la forte impédance électrique du *stratum corneum.* Des électrodes micro-aiguilles capables de percer celui-ci permettent d'atteindre des impédances de mesure de plusieurs ordres de grandeur plus basses. Cependant, à cause de leurs matériaux rigides et non-biodégradables, ces dispositifs sont très sensibles aux mouvements parasites et apportent un danger pour le patient en cas de casse. Des micro-aiguilles en matériaux biocompatibles existent mais soit ne sont pas utilisées dans le cadre de la mesure électrique, soit sont trop complexes à employer et fabriquer pour un déploiement commercial.

Les inventeurs se sont donc fixé pour but de proposer un dispositif apte à réaliser des mesures électriques sans présenter les inconvénients des électrodes micro-aiguilles de l'art antérieur, ce dispositif devant être facile à préparer et à mettre en oeuvre.

### EXPOSÉ DE L'INVENTION

La présente invention permet d'atteindre le but que se sont fixé les inventeurs puisque ces derniers ont mis au point un dispositif du type patch dont une première surface destinée à être appliquée sur la peau d'un patient est nano-structurée et/ou micro-structurée et dont une seconde surface différente de la première est munie d'une connectique.

Par ailleurs, ce patch en un matériau comprenant un polymère réticulé biocompatible est non conducteur mais une fois appliqué sur la peau et une fois que les nanostructures ou les microstructures qu'il comprend ont percé le *stratum corneum,* le polymère réticulé se gonfle de liquide interstitiel, le patch devenant, de par la présence et la nature chimique de ce liquide, conducteur de l'électricité. Lorsqu'il est gorgé de liquide interstitiel, ce patch est comparable à une électrode et peut être utilisé pour effectuer des mesures électriques.

Par rapport aux dispositifs commerciaux classiques tels que les électrodes humides, les apports principaux du dispositif selon l'invention sont :
- l'amélioration de la qualité de la mesure électrique, mesurée par le rapport signal sur bruit qui lui-même est inversement proportionnel à l'impédance électrique observée lors de la mesure ;
- l'amélioration de la sécurité du patient, de par la biocompatibilité des matériaux employés, leur capacité à se dégrader en milieu physiologique en quelques semaines, leur usage unique ainsi que la simplicité d'usage ne demandant pas de réaliser de gestes médicaux spécialistes ;
- les matériaux et le procédé de fabrication restent simples, peu coûteux et permettent de fabriquer des patches limitant l'inconfort du patient ainsi que les artéfacts de mesure lors de mouvements permettant ainsi son emploi en tant que capteur portable longue durée de l'ordre de quelques jours.

Plus particulièrement, la présente invention concerne un dispositif du type électrode comprenant
i) un patch ayant une première et une deuxième faces opposées,
   ladite première face présentant des nano-structures et/ou des micro-structures,
   ledit patch, lesdites nano-structures et lesdites micro-structures étant en un matériau comprenant au moins un polymère réticulé, non-conducteur, et
ii) un élément de reprise de contact électrique, appliqué sur ou intégré au niveau de ladite deuxième face.

Parmi les éléments essentiels du dispositif selon l'invention, on trouve (α) les microstructures et/ou nanostructures que le patch présente, (β) la nature chimique du patch et de ces micro-structures et/ou nano-structures et (γ) l'élément de reprise de contact électrique.

Le patch compris dans le dispositif selon l'invention peut présenter une forme et une taille quelconques. Ces dernières pourront être choisies en fonction de la morphologie de la zone sur laquelle le dispositif selon l'invention doit être appliqué. Typiquement, il peut présenter une forme de carré, de rectangle, de disque, d'ovale ou d'ellipse. La longueur la plus grande de ces formes est avantageusement comprise entre 2 mm et 15 cm, notamment entre 4 mm et 10 cm et, en particulier, entre 6 mm et 2 cm. De plus, le patch mis en oeuvre dans l'invention i.e. avec les nano-structures et/ou les micro-structures présente avantageusement une épaisseur comprise entre 200 µm et 1,5 cm et notamment entre 1 mm et 1 cm.

La surface du patch mis en oeuvre dans l'invention devant être apposée ou appliquée sur la peau présente des nano-structurations ou des micro-structurations comme, par exemple, des micro-aiguilles ou des micro-piliers. Ces nano-structurations ou micro-structurations permettent un ancrage mécanique du patch au niveau de la peau. A titre d'exemples de telles structurations, on peut citer des micro-aiguilles capables de traverser le *stratum corneum* et d'atteindre les couches sous-cutanées, sur une profondeur de 100 µm à 2000 µm, notamment de 200 µm à 1500 µm et, en particulier, de 300 µm à 1200 µm. Ainsi, la surface du patch mis en oeuvre dans l'invention devant être apposée ou appliquée sur la peau présente des micro-aiguilles dont la hauteur moyenne est comprise entre 150 µm et 3000 µm, notamment entre 250 µm et 2000 µm et, en particulier, entre 300 µm et 1500 µm. Typiquement, la largeur moyenne à la base de ces micro-aiguilles est comprise entre 50 µm et 1000 µm, notamment entre 100 µm et 800 µm et, en particulier, entre 200 µm et 600 µm et/ou l'espace moyen inter-aiguilles est compris entre 150 µm et 3000 µm, notamment entre 250 µm et 2000 µm et, en particulier, entre 300 µm et 1500 µm.

Le patch et les nano-structures et/ou les micro-structures qu'il présente sont en un matériau comprenant au moins un polymère réticulé, non-conducteur. Il est clair que le patch et les nano-structures et/ou les micro-structures qu'il présente sont réalisés dans le même matériau. Par ailleurs, le patch mis en oeuvre dans l'invention ne présente ou n'est associé à aucune nano-structure ou aucune micro-structure du type micro-aiguille en un matériau métallique. De plus, la surface externe des nano-structures et des micro-structures que peut présenter le patch mis en oeuvre dans l'invention n'est pas métallisée i.e. ne présente aucun revêtement en métal.

Le ou les polymère(s) réticulé(s) non-conducteur(s) compris dans le matériau mis en oeuvre dans le cadre de l'invention présente(nt) la capacité de se gonfler de liquide interstitiel lorsque les nano-structures et/ou les micro-structures sont mises en contact avec les couches sous-cutanées. Ce type de polymère connu sous l'expression « polymère super-absorbant » (ou SAP) est capable, à l'état sec, d'absorber spontanément au moins 2 fois (soit 2 g de liquide aqueux absorbé par gramme de polymère absorbant), au moins 5 fois, au moins 10 fois, avantageusement au moins 20 fois son poids de liquide aqueux, en particulier d'eau et notamment d'eau distillée. Par « absorption spontanée », on entend un temps d'absorption inférieur ou égal à 1h30 et notamment inférieur ou égal à 1h. Ces caractéristiques d'absorption de liquide aqueux s'entendent dans les conditions normales de température (25°C) et de pression (760 mm Hg soit 100000 Pa).

Par ailleurs, le ou les polymère(s) réticulé(s) non-conducteur(s) compris dans le matériau mis en oeuvre dans le cadre de l'invention est/sont typiquement biodégradable(s). En effet, comme les nano-structures et/ou les micro-structures que présente le patch sont introduites dans le corps du patient, il convient que le ou les polymère(s) réticulé(s) non-conducteur(s) puisse(nt) être facilement dégradé(s) sans générer de résidu toxique si certaines des nano-structures et/ou des micro-structures se cassaient et restaient dans les couches sous-cutanées.

Tout type de polymères réticulés, non-conducteur(s), hydrophiles et connus de l'homme du métier est utilisable dans le cadre de la présente invention. Ce ou ces polymère(s) réticulé(s) non-conducteur(s) est/sont choisi(s) dans le groupe constitué par les polymères réticulés synthétiques, les biopolymères réticulés et leurs mélanges. Par « mélange », on entend (i) un mélange d'au moins deux polymères réticulés synthétiques, (ii) un mélange d'au moins deux biopolymères réticulés et (iii) un mélange d'au moins un polymère réticulé synthétique et d'au moins un biopolymère réticulé. Dans un mélange, au moins deux polymères réticulés différents peuvent être non liés l'un à l'autre ou liés l'un à l'autre via des liaisons covalentes et/ou des liaisons non covalentes.

Dans un mode de réalisation particulier, le au moins un polymère réticulé non-conducteur est un polymère réticulé synthétique. Ce dernier est notamment choisi dans le groupe constitué par les polyéthylène glycols, les acides polyacryliques (PAA), les alcools polyvinyliques (PVA) et leurs mélanges.

Avantageusement, le au moins un polymère réticulé non-conducteur compris dans le matériau mis en oeuvre dans le cadre de l'invention est un biopolymère réticulé. Par « biopolymère », on entend un polymère produit par un organisme vivant ou un dérivé de celui-ci, ou est une version synthétique de ce polymère produite par des voies chimiques abiotiques.

Typiquement, les biopolymères utilisables pour préparer les biopolymères réticulés mis en oeuvre dans l'invention comprennent, sans s'y limiter, des protéines et/ou des polysaccharides.

En particulier, les biopolymères réticulés mis en oeuvre dans l'invention sont obtenus à partir de biopolymères choisis dans le groupe constitué par les amidons (y compris l'amylose et/ou l'amylopectine), les chitosanes, les hémicelluloses, les glycanes, les lignines, les celluloses, les chitines, les alginates, les dextranes, les pullanes, les polyhydroxyalcanoates, les fibrines, les cyclodextrines, le collagène, la gélatine, les fibroïnes de soie, la pectine, les glycosaminoglycanes, les acides polylactiques, les acides polyuroniques, un sel de ceux-ci, une forme substituée de ceux-ci, un sel de celle-ci et leurs mélanges.

Plus particulièrement, les biopolymères réticulés mis en oeuvre dans l'invention sont obtenus à partir de biopolymères choisis dans le groupe constitué par la méthylcellulose, l'éthylcellulose, la n-propylcellulose, l'hydroxyéthylcellulose, l'hydroxy-n-propylcellulose, l'hydroxy-n-butylcellulose, l'hydroxypropylméthylcellulose, l'éthylhydroxyéthylcellulose, la carboxyméthylcellulose (CMC), l'amidon de maïs, l'hydroxypropylamidon, le carboxyméthylamidon, le sulfate de dextrane, le phosphate de dextrane, du dextran acrylé, du dextran méthacrylé, le diéthylaminodextrane, l'héparine, le hyaluronane, la chondroïtine, le sulfate de chondroïtine, le sulfate d'héparane, l'acide polyglucuronique, l'acide polymanuronique, l'acide polygalacturonique et l'acide polyarabinique.

Pour des applications dans lesquelles le dispositif selon l'invention doit être maintenu sur un patient plus de trois jours, des polymères réticulés tels que de la CMC dont la masse molaire est supérieure à 250 kg/mol et notamment supérieure ou égale à 300 kg/mol seront préférés.

Dans un mode de réalisation particulier, le matériau du patch mis en oeuvre dans l'invention ne comprend qu'un ou plusieurs polymère(s) réticulé(s) non-conducteur(s) tel(s) que précédemment défini(s). En d'autres termes le matériau du patch est constitué d'un ou plusieurs polymère(s) réticulé(s) non-conducteur(s) tel(s) que précédemment défini(s).

Dans un autre mode de réalisation particulier, le matériau du patch mis en oeuvre dans l'invention est constitué de ou comprend au moins un polymère réticulé non-conducteur tel que précédemment défini et au moins un autre additif. Il est clair que ce ou ces additif(s) doi(ven)t être biodégradable(s) et biocompatible(s). Typiquement, ce ou ces additif(s) est/sont choisi(s) dans le groupe constitué par les plastifiants, les sels, les conservateurs et les colorants.

Le ou les plastifiant(s) que peut comprendre le matériau du patch mis en oeuvre dans l'invention permet(tent) de conférer au patch des propriétés de flexibilité ajustables afin de se conformer au mieux à la forme de la peau et de mieux y adhérer. Typiquement, ce ou ces plastifiant(s) est/sont choisi(s) dans le groupe constitué par les glycols présentant une masse moléculaire comprise entre 100 g/mol et 20 000 g/mol tels que le polyéthylène glycol (PEG) et le propylène glycol ; la lécithine ; l'acide citrique ; des huiles végétales comme de l'huile d'olive ou de l'huile de ricin ; des esters d'acide gras tels que le myristate d'isopropyle, le laurate d'hexyle, le sébacate de diéthyle, le sébacate de diisopropyle et le sébacate de dibutyle ; des amines telles que la monoéthanolamine, la diéthanolamine, la triéthanolamine, la monisopropanolamine et la triéthylenetetramine et des tensioactifs tels que des tensioactifs non-ioniques. A titre d'exemples de tensioactifs non-ioniques utilisables comme plastifiants dans le cadre de la présente invention, on peut citer les alcools polyéthoxylés, les phénols polyéthoxylés, les oléates, les laurates et leurs mélanges. Avantageusement, le plastifiant que peut comprendre le matériau du patch mis en oeuvre dans la présente invention est du polyéthylène glycol (PEG) et notamment du PEG dont la masse moléculaire est comprise entre 6 kg/mol et 20 kg/mol.

En d'autres termes, le dispositif du type électrode objet de l'invention comprend
(i) un patch ayant une première et une deuxième faces opposées, ladite première face présentant des nano-structures et/ou des micro-structures,
   ledit patch, lesdites nano-structures et lesdites micro-structures étant en un matériau constitué d'un ou de plusieurs polymère(s) réticulé(s) non-conducteur(s) et éventuellement d'au moins un additif choisi dans le groupe constitué par les plastifiants, les sels, les conservateurs et les colorants, et
   la surface externe des nano-structures et des micro-structures n'étant pas métallisée,
(ii) un élément de reprise de contact électrique, appliqué sur ou intégré au niveau de ladite deuxième face.

Lorsque le matériau du patch mis en oeuvre dans la présente invention comprend au moins un autre additif que le au moins polymère réticulé non-conducteur, la masse totale dudit au moins un polymère réticulé non-conducteur est supérieure ou égale à 75%, notamment supérieure ou égale à 80% et, en particulier, supérieure ou égale à 85% par rapport à la masse sèche totale du patch.

Enfin, le dispositif selon l'invention comprend un élément de reprise de contact électrique, appliqué sur ou intégré au niveau de la deuxième face du patch i.e. la face opposée à la face présentant une nano-structuration et/ou une micro-structuration définie comme la première face. Cet élément de reprise de contact électrique permet de coupler électriquement le dispositif selon l'invention à un système de mesure tel qu'un potentiostat.

Tout élément de reprise de contact électrique connu de l'homme du métier est utilisable dans le cadre de la présente invention. Typiquement, cet élément de reprise de contact est choisi dans le groupe constitué par une électrode ; une couche conductrice comme une couche métallique ; un ruban conducteur comme un ruban métallique ; un adhésif conducteur; un fil conducteur comme un film métallique et une de leurs combinaisons. Par « métallique », on entend en un matériau choisi parmi le cuivre, le platine, le cobalt, l'or, le titane, le palladium, le chrome, l'aluminium et leurs alliages.

La présente invention concerne également un procédé de préparation d'un tel dispositif. Ce procédé comprend les étapes consistant à :
a) préparer une solution aqueuse contenant au moins un polymère réticulé non-conducteur ou au moins un de ses précurseurs et éventuellement au moins un additif ;
b) appliquer ladite solution préparée à l'étape a) sur un support ayant une forme et une géométrie, adaptées pour (permettant de) conférer, au patch obtenu après séchage de la solution aqueuse, une face nano-structurée et/ou micro-structurée ;
c) sécher ladite solution puis éventuellement soumettre ledit au moins un des précurseurs à des conditions permettant l'obtention dudit au moins un polymère réticulé non-conducteur moyennant quoi un patch est obtenu ;
le procédé comprenant, préalablement ou après cette étape c), une étape d'application sur ou d'intégration au niveau de la surface de la solution obtenue après l'étape b) ou de la face du patch à l'opposé de la face micro-structurée ou nano-structurée, d'un élément de reprise de contact électrique.

Tout ce qui a été précédemment décrit pour le patch du dispositif selon l'invention en termes de polymère(s) réticulé(s) non-conducteur(s) et éventuel(s) additif(s) s'applique *mutatis mutandis* à la solution aqueuse préparée lors de l'étape a).

Dans un mode de réalisation avantageux, cette solution peut toutefois ne pas contenir un ou des polymère(s) réticulé(s) non-conducteur(s) mais un ou plusieurs des précurseurs de celui-ci/ceux-ci. Par « précurseur d'un polymère réticulé non-conducteur », on entend typiquement le polymère non réticulé non-conducteur et éventuellement au moins un agent de réticulation.

Dans ce mode de réalisation, toute technique, chimique et/ou physique, connue de l'homme du métier pour obtenir un polymère réticulé à partir du précurseur non réticulé est utilisable.

A titre d'exemples de telles techniques chimiques, on peut citer des réactions chimiques biocompatibles dans l'eau comme une réaction d'estérification impliquant une fonction acide et une fonction alcool, une réaction impliquant deux fonctions thiols ou une fonction thiol et une fonction maléimide, une réaction impliquant une fonction aldéhyde/cétone et une fonction oxyamine, un couplage peptidique, une réaction de Diels-Alder et une réaction de chimie click (sans catalyseur au cuivre).

A noter que le polymère non réticulé non-conducteur peut être modifié pour introduire, dans sa structure, une ou plusieurs fonctions réactives participant à la réticulation. Avantageusement, l'agent de réticulation mis en oeuvre dans le cadre de l'invention est un acide polycarboxylique choisi parmi les acides dicarboxyliques en C4-C12, les acides tricarboxyliques et les acides tétracarboxyliques. En particulier, l'acide polycarboxylique utilisable dans l'invention est choisi dans le groupe constitué par l'acide malonique, l'acide malique, l'acide maléique, l'acide succinique, l'acide glutarique, l'acide adipique, l'acide citrique, l'acide isocitrique, l'acide aconitique, l'acide propane-1,2,3-tricarboxylique et l'acide 2,3,3',4'-biphényltétracarboxylique. Plus particulièrement, l'agent de réticulation est l'acide citrique.

En variante, l'agent de réticulation peut être un photo-initiateur. Ce dernier est apte à entraîner la photo-réticulation d'un polymère photosensible tel qu'un polysaccharide acrylé ou méthacrylé comme le dextran acrylé ou méthacrylé. A titre d'exemples particuliers de tels photo-initiateurs, on peut citer le phényl-2,4,6-triméthylbenzoylphosphinate de lithium (LAP) ou le 2-hydroxy-4'-(2-hydroxyéthoxy)-2-méthylpropiophénone (également connu sous la dénomination « Irgacure 2959 »).

L'étape a) du procédé selon l'invention consiste à mélanger ensemble les différents composants de la solution, à savoir soit au moins un polymère réticulé non-conducteur, soit au moins un de ses précurseurs et éventuellement au moins un additif tel que précédemment décrit, dans un solvant aqueux de façon à obtenir une solution aqueuse dans laquelle sont dissous soit au moins un polymère réticulé non-conducteur, soit au moins un de ses précurseurs et éventuellement au moins un additif. Les polymères réticulés non-conducteurs ou leurs précurseurs et éventuels additifs peuvent être ajoutés en bloc, par groupe ou les uns après les autres.

Dans un mode de réalisation particulier, l'étape a) consiste à :
- ajouter, à un solvant aqueux comme de l'eau ou de l'eau distillée, un ou des polymère(s) non réticulé(s) non-conducteur(s) et éventuellement un ou plusieurs additif(s) puis mélanger l'ensemble, sous agitation notamment générée par une roue tournante, pendant une durée et à une température adaptées pour (permettant d') obtenir un mélange homogène. A titre d'exemple illustratif, lorsque le polymère non réticulé non-conducteur est de la CMC, cette sous-étape présente une durée comprise entre 24 h et 72 h et notamment de l'ordre de 48 h (i.e. 48 h ± 6 h) et est réalisée à une température supérieure ou égale à la température ambiante (i.e. 23°C ± 5°C) et inférieure à 80°C.
- ajouter à la solution aqueuse contenant un ou des polymère(s) non réticulé(s) non-conducteur(s) et éventuellement un ou plusieurs additif(s) ainsi obtenue, au moins un agent de réticulation puis mélanger l'ensemble, sous agitation notamment générée par un vortex ou un barreau aimanté, pendant une durée et à une température adaptées pour (permettant d') obtenir un mélange homogène. A titre d'exemple illustratif, lorsque le polymère non réticulé non-conducteur est de la CMC, cette sous-étape présente une durée comprise entre 15 min et 45 min et notamment de l'ordre de 30 min (i.e. 30 min ± 5 min) et est réalisée à une température inférieure à 40°C, notamment à une température comprise entre 20°C et 35°C et, en particulier, à environ 30°C (i.e. 30°C ± 3°C).

Le mélange homogène ainsi obtenu est une solution aqueuse contenant un ou des polymère(s) non réticulé(s) non-conducteur(s), au moins un agent de réticulation et éventuellement un ou plusieurs additif(s).

L'agent de réticulation est typiquement utilisé en une quantité en masse comprise entre 5% et 20% et, en particulier, de 10% par rapport à la masse du ou des polymère(s) non réticulé(s) non-conducteur(s).

Il est possible de soumettre la solution aqueuse obtenue à l'issue de l'étape a) à une sonication afin d'éliminer, avant son dépôt sur le support, les bulles d'air qu'elle pourrait contenir.

Le support sur lequel est déposée, lors de l'étape b), la solution aqueuse préparée lors de l'étape a) a une forme et une géométrie, adaptées pour (permettant de) conférer, au patch obtenu après séchage de la solution aqueuse, une forme nano-structurée et/ou micro-structurée.

A cet effet, le support mis en oeuvre lors de l'étape b) peut être un moule et notamment un moule en silicone. Un tel moule en silicone peut être facilement obtenu par différentes méthodes connues de l'homme du métier. Ainsi, l'étape b) du procédé selon l'invention consiste à remplir ce moule avec la solution aqueuse préparée lors de l'étape a). Le remplissage du moule peut être effectué
- en soumettant le moule dans lequel la solution aqueuse a été déposée à une centrifugation comme une centrifugation à vitesse comprise entre 500 et 6000 rotations par minute (rpm) et notamment de l'ordre de 4000 rpm (i.e. 4000 rpm ± 500 rpm), pendant une durée comprise entre 5 min et 1 h et notamment de l'ordre de 30 min (i.e. 30 min ± 10 min) ou
- en appliquant une surpression comprise entre 100 Pa et 10 MPa sur la solution aqueuse introduite dans le moule, par le biais d'un couvercle ayant une forme adaptée pour ne pas endommager la reprise de contact; en particulier, ce couvercle n'est pas adhérent à la solution aqueuse, par exemple, il est en Téflon ou silicone ou comporte un revêtement pour la face en contact avec la solution en Téflon ou silicone, ou
- en appliquant un vide ou une succession de vides de puissance décroissante par le dessous du moule. Dans un mode de réalisation particulier, cette succession de vides consiste à appliquer un vide de l'ordre de 800 mbar (i.e. 800 mbar ± 50 mbar) pendant 10 min, puis un vide de l'ordre de 100 mbar (i.e. 100 mbar ± 20 mbar) pendant 10 min et enfin un vide de l'ordre de 20 mbar (i.e. 20 mbar ± 5 mbar) pendant 100 min ou
- en plaçant le moule tout entier dans une enceinte sous vide de l'ordre de 20 mbar (i.e. 20 mbar ± 5 mbar) pendant une durée de l'ordre de 5 min (i.e. 5 min ± 1 min). Ce dernier procédé a l'avantage d'être plus facilement collectif (seule limitation : la taille de l'enceinte) et plus court, tout en nécessitant moins de contraintes par rapport au moule.

Une telle étape b) avec application d'un ou plusieurs vide(s) et/ou d'une ou plusieurs surpression(s) présente plusieurs avantages notamment par rapport à une méthode classique de dépôt par centrifugation : elle est transposable à l'échelle industrielle et compatible avec un procédé collectif de fabrication.

Typiquement, l'étape b) du procédé selon l'invention est réalisée à une température inférieure à 35°C, notamment à une température comprise entre 15°C et 30°C et, en particulier, à température ambiante.

L'étape c) du procédé selon l'invention consiste à soumettre le support sur lequel a été appliquée la solution aqueuse préparée à l'étape a) à des conditions permettant le séchage de cette solution puis éventuellement à des conditions permettant la réticulation du ou des polymère(s) précurseur(s) afin d'obtenir un ou des polymère(s) réticulé(s) non-conducteur(s) et ainsi un patch tel que compris dans le dispositif selon l'invention.

Typiquement, le séchage lors de l'étape c) est effectué dans une étuve, à une température comprise entre la température ambiante et 50°C et notamment de l'ordre de 30°C (i.e. 30°C ± 5°C). La durée du séchage de l'étape c) sera choisie en fonction de la masse de la solution aqueuse déposée. Avantageusement, cette durée est comprise entre 12 h et 60 h et notamment de l'ordre de 24 h (i.e. 24 h ± 6 h).

En fonction du type de réticulation utilisée pour permettre la formation d'un ou des polymère(s) réticulé(s) non-conducteur(s) lorsque la solution aqueuse préparée lors de l'étape a) du procédé contient un ou plusieurs précurseur(s) de ce ou ces polymère(s) réticulé(s) non-conducteur(s), l'homme du métier saura déterminer, sans effort inventif, les conditions à mettre en oeuvre pour former ce ou ces polymère(s) réticulé(s) non-conducteur(s).

Dans un mode de réalisation particulier, les conditions permettant la formation d'un ou des polymère(s) réticulé(s) non-conducteur(s) lorsque la solution aqueuse préparée lors de l'étape a) du procédé contient un ou plusieurs précurseur(s) de ce ou ces polymère(s) réticulé(s) non-conducteur(s) consistent à maintenir le moule, dans l'étuve dans laquelle il a été soumis à un séchage, et à amener la température de l'étuve à une température comprise entre 50°C et 90°C notamment de l'ordre de 80°C (i.e. 80°C ± 5°C) et à maintenir cette température pendant une durée comprise entre 4 h et 60 h et notamment de l'ordre de 24 h (i.e. 24 h ± 6 h).

Dans un autre mode de réalisation particulier impliquant une photo-réticulation, le support sur lequel la solution aqueuse a été déposée puis séchée sera soumis à une irradiation à une longueur d'ondes adaptée au photo-initiateur que contient la solution aqueuse préparée lors de l'étape a). A titre d'exemple particulier, lorsque le photo-initiateur utilisé est du LAP ou de l'Irgacure 2959, une irradiation UV-vis à 405 nm typiquement avec une puissance de 1 à 100 mW/cm² pendant une durée de 30 s à 10 min sera mise en oeuvre lors de l'étape c).

Une fois le patch obtenu après l'étape c) de séchage et d'éventuelle réticulation, il est facilement récupérable à partir du support notamment par démoulage.

Préalablement à cette étape de séchage et d'éventuelle réticulation, il est possible d'intégrer un élément de reprise de contact électrique tel que précédemment défini au niveau de la surface de la solution aqueuse contenant au moins un polymère réticulé non-conducteur ou au moins un de ses précurseurs et éventuellement au moins un additif moyennant quoi, après l'étape de séchage et d'éventuelle réticulation, cet élément de reprise électrique se retrouve intégré au niveau de la face opposée à la face du patch mise en contact avec la peau.

En variante, suite à l'étape c) et avant ou après ce démoulage, il est possible d'associer, au patch, un élément de reprise de contact électrique tel que précédemment défini sur la face opposée à la face du patch mise en contact avec la peau. Cette variante permet d'obtenir un patch sur une face duquel un élément de reprise de contact électrique est appliqué.

Le dispositif ainsi préparé peut être conservé plusieurs semaines, i.e. au moins deux, au moins trois semaines ou encore au moins cinq semaines, à l'abri de l'humidité. Cette absence d'humidité peut être obtenue grâce à un sachet de desséchant et/ou sous vide et/ou sous gaz inerte comme de l'argon. Cette conservation peut être réalisée à une température comprise entre 4°C et 60°C en fonction du ou des polymère(s) réticulé(s) compris dans le matériau du patch. Dans certaines formes de mise en oeuvre, la conservation se fait à température ambiante (i.e. 23°C ± 5°C) sous desséchant ou à une température de l'ordre de 50°C (i.e. 50°C ± 5°C).

La présente invention concerne l'utilisation d'un dispositif tel que précédemment défini comme électrode pour un électrocardiogramme (ECG), pour un électroencéphalogramme (EEG), pour un électromyogramme (EMG), pour un électro-oculogramme (EOG), pour un électrogastrogramme (EGG), pour une tomographie d'impédance électrique (EIT pour « Electrical impédance Tomography ») et pour une bioimpédance.

En d'autres termes, la présente invention concerne l'utilisation comme électrode pour un électrocardiogramme (ECG), pour un électroencéphalogramme (EEG), pour un électromyogramme (EMG), pour un électro-oculogramme (EOG), pour un électrogastrogramme (EGG), pour une tomographie d'impédance électrique (EIT pour « Electrical impédance Tomography ») et pour une bioimpédance, d'un dispositif de type électrode comprenant :
i) un patch ayant une première et une deuxième faces opposées, ladite première face présentant des nano-structures et/ou des micro-structures,
   ledit patch, lesdites nano-structures et lesdites micro-structures étant en un matériau comprenant au moins un polymère réticulé non-conducteur,
   la surface externe des nano-structures et des micro-structures n'étant pas métallisée et
ii) un élément de reprise de contact électrique, appliqué sur ou intégré au niveau de ladite deuxième face.

En effet, le dispositif selon l'invention présente, une fois que les nano-structures et/ou les micro-structures du patch ont traversé le *stratum corneum* de la peau d'un patient et se trouvent dans les couches sous-cutanées, une impédance minimale comme illustré dans la partie expérimentale ci-après. Par « impédance minimale », on entend une impédance inférieure ou égale à 10 kΩ. Une telle impédance permet une meilleure conductivité et une meilleure conduite des signaux électriques représentant des paramètres biologiques à travers la peau d'un patient. Par « patient », on entend aussi bien un humain qu'un animal non humain comme un chat, un chien ou un cheval.

Pour rappel, ce dispositif est à usage unique puisque, une fois en contact avec le liquide interstitiel, le ou les polymère(s) réticulé(s) non-conducteur(s) compris dans le matériau du patch se gonfle(nt) et ramolli(ssen)t empêchant toute réutilisation.

Par ailleurs, le dispositif selon l'invention est facile d'utilisation. En effet, il suffit d'appliquer la face du patch comprenant les nano-structures et/ou les micro-structures sur la peau du patient et d'appuyer, par exemple avec le pouce ou un tampon, sur la face opposée afin que les nano-structures et/ou les micro-structures du patch traversent le *stratum corneum* de la peau du patient. En variante, on peut envisager d'associer le dispositif à un applicateur au niveau de la face opposée à la face du patch comprenant les nano-structures et/ou les micro-structures et d'utiliser cet applicateur pour disposer la face du patch comprenant les nano-structures et/ou les micro-structures sur la peau du patient et faire traverser le *stratum corneum* de la peau du patient par les nano-structures et/ou les micro-structures du patch. Pour ce faire, l'applicateur peut comprendre un système à ressort.

La présente invention concerne enfin un système porté pour le suivi en ambulatoire d'un patient, comprenant un dispositif tel que précédemment défini. Dans un tel système notamment utile pour surveiller les patients à risque à leur domicile, sont mises à profit la capacité du dispositif selon l'invention et notamment du patch qu'il comprend à être biodégradable et sa capacité à résister aux artéfacts de mouvement. Ce système porté peut comprendre, outre le dispositif selon l'invention, tout élément additionnel classiquement utilisé avec des systèmes portés comprenant des électrodes extra-dermiques humides. Dans ce système porté, le dispositif selon l'invention peut être connecté, via sa reprise de contact électrique, à tout dispositif permettant une mesure ECG, EEG... classique. De même, le dispositif selon l'invention peut cependant être déporté des dispositifs de mesure à l'aide de câbles électriques.

D'autres caractéristiques et avantages de la présente invention apparaîtront encore à l'homme du métier à la lecture des exemples ci-dessous donnés à titre illustratif et non limitatif, en référence aux figures annexées.

### BRÈVE DESCRIPTION DES DESSINS

La Figure 1 est une représentation schématisée des différentes étapes de préparation d'un dispositif selon l'invention.
La Figure 2 présente le principe de fonctionnement d'un dispositif selon l'invention du type électrode micro-aiguilles à gonflement.
La Figure 3 présente le module de l'impédance à 10 Hz en fonction du temps pour trois configurations d'électrodes de test, à savoir électrode du commerce (ECG) sur épiderme, électrode micro-aiguille (MA) selon l'invention sur épiderme et électrode du commerce (ECG) sur couches sous-cutanées (i.e. après ablation de l'épiderme).
La Figure 4 présente la prise de masse du matériau CMC de masse molaire variable (95 kDa, 250 kDa, 395 kDa et 725 kDa) à taux de réticulant fixe (acide citrique 7,5%) en fonction du temps d'immersion dans du PBS à 37°C.
La Figure 5 présente la masse relative d'un gel CMC + 10% polyéthylène glycol (par rapport à la masse de CMC) (PEG BioUltra 3,350 [Sigma-Aldrich]) à différents taux de réticulation (acide citrique (CA) à 0%, 7,5% et 15%) en fonction du temps d'immersion dans du PBS à 37°C.
La Figure 6 présente, en partie gauche, des microscopies des électrodes microaiguilles CMC (95 kDa) réticulées à différents taux d'acide citrique (CA 10% et CA 30%) avant et après compression orthogonale à 50 N et, en partie droite, la pente des courbes force-déplacement associées (3 échantillons de chaque).
La Figure 7 présente la masse relative de gels CMC/CA (acide citrique à 7,5%) avec 10% de PEG d'une masse molaire variable (3 kDa, 6 kDa et 20 kDa) en fonction du temps d'immersion dans du PBS à 37°C.

### EXPOSÉ DÉTAILLÉ DE MODES DE RÉALISATION PARTICULIERS

### I. Procédé de préparation d'un dispositif selon l'invention.

### 1.1. Matériaux utilisés.

Les matériaux utilisés pour la préparation du moule et du patch sont :
- du silicone Sylgard 184 + réticulant thermique [Sigma-Aldrich]
- de la carboxyméthylcellulose 7LPEP 95 kg/mol (CMC) [Ashland] et
- de l'acide citrique >= 99.5% [Sigma-Aldrich]
- du poly(éthylène glycol) linéaire terminaison hydroxyle 3350 g/mol BioUltra 3.350 [Sigma-Aldrich].

### 1.2. Etapes du procédé de préparation.

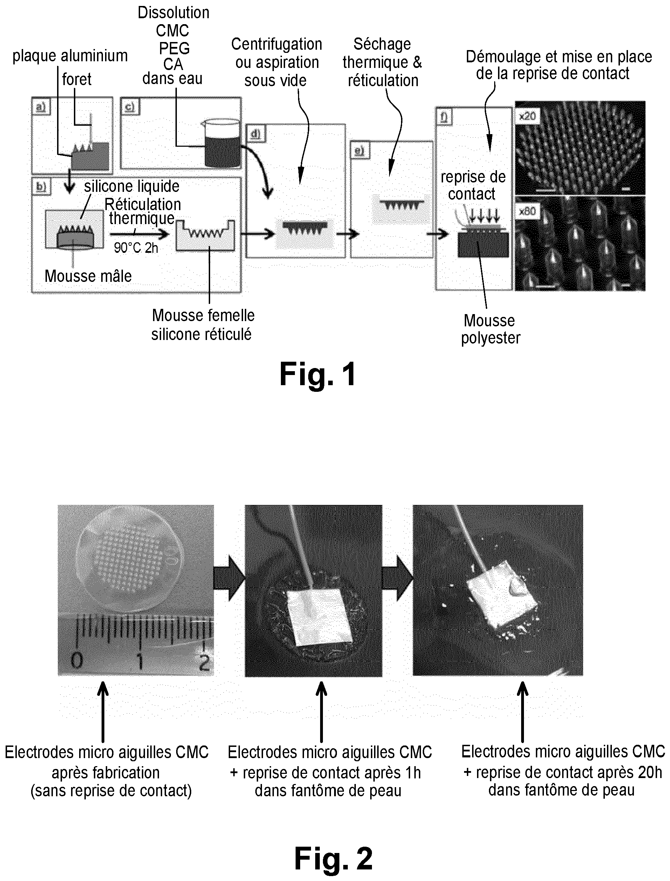

La Figure 1 illustre les étapes du procédé. Ces dernières consistent en :

### i. Fabrication d'un contre-moule

Étape a : Un moule mâle en aluminium est micro-usiné selon la forme désirée des micro-aiguilles.
Étape b : Un contre-moule femelle est réalisé à l'aide de silicone liquide (en proportion 10:1 (masse/masse)) réticulé en étuve 90°C pendant 2 h.

### ii. Préparation de la solution de polymère

Étape c : La CMC est dissoute dans l'eau distillée (proportion 4:100 (masse/masse)) à l'aide d'une roue tournante à une vitesse de 5 rotations par minute (rpm) durant 48 h.

L'acide citrique est ajouté à une proportion de 10% de la masse de CMC. La solution est agitée à l'aide d'un barreau aimanté, 30 min, à 1400 rpm et à 30°C. La solution est ensuite dégazée au bain à ultrasons pendant 15 min.

### iii. Remplissage du moule

Étape d : Le moule silicone est rempli à l'aide d'une pipette de 600 µL de la solution de polymère préparée précédemment. Ce moule est ensuite placé en centrifugeuse à 4000 rpm, à 20°C, pendant 30 min.

### iv. Séchage du solvant et réticulation du polymère

Étape e : Après centrifugation, le moule est placé en étuve à 30°C pendant 24 h puis à 80°C pendant 24 h. Ces étapes permettent successivement le séchage de l'eau et la réticulation du polymère, respectivement.

### v. Démoulage et application de la reprise de contact

Étape f : Le patch MN sec est démoulé manuellement du moule silicone.

Une reprise de contact, constituée d'un ruban adhésif double-face conducteur en aluminium auquel est soudé un fil électrique est appliqué à l'aide d'une force de 3N sur la face arrière du patch micro-aiguilles lui-même placé sur une mousse durant l'opération afin d'éviter sa dégradation.

### II. Preuve de concept.

### II.1. Fantôme de peau.

Afin de réaliser une preuve de concept des dispositifs selon l'invention, un fantôme de peau ayant des propriétés électriques représentatives de celle-ci a été développé. Ce fantôme est composé de deux couches distinctes :
- la première couche représentant l'épiderme, est composée de la même silicone que décrit précédemment et est d'une épaisseur d'une centaine de micromètres. Cette couche est isolante électriquement.
- la seconde couche représentant les couches sous-cutanées (derme et hypoderme), est composée d'un mélange de gélatine (type A, force de gel 300) [Sigma-Aldrich] (24% masse/masse) et d'agar [Millipore] (1% masse/masse) dissous dans du PBS 1X et d'une épaisseur de 20 mm. Le PBS 1X possède des propriétés électriques proches du liquide interstitiel et représente raisonnablement celui-ci dans ce contexte.

La Figure 2 présente le principe de fonctionnement du dispositif selon l'invention, une fois l'élément de reprise de contact appliqué et après mise en contact avec le fantôme de peau tel que décrit ci-dessus pendant différentes durées (1 h et 20 h).

### II.2. Performances électriques.

Les performances électriques du dispositif selon l'invention du type électrodes micro-aiguilles (MA) ont été mesurées en comparaison avec des électrodes ECG commerciales [Softrace, Conmed corporation].

Dans cette optique, une électrode ECG commerciale de référence a été invariablement placée directement sur les couches sous-cutanées du modèle de peau. Une seconde électrode, celle faisant l'objet du test, a été placée sur l'épiderme ou les couches sous-cutanées.

Le module de l'impédance mesuré à 10 Hz est inversement proportionnel au rapport signal sur bruit d'une mesure électrique on-vivo (le plus souvent réalisé entre 1 et 1000 Hz); il représente donc une figure de mérite pertinente pour les applications visées.

Trois configurations d'électrodes ont été testées :
- la première où une électrode commerciale ECG est placée directement sur l'épiderme **→** Représentatif d'une mesure sans préparation du patient.
- une seconde où une électrode commerciale est placée directement sur les couches sous-cutanées **→** Représentatif d'une mesure après ablation de l'épiderme.
- une troisième où une électrode micro-aiguilles selon l'invention est insérée dans l'épiderme via appui du doigt -+ Représentatif d'une utilisation classique d'une telle électrode - sans préparation préalable de la peau.

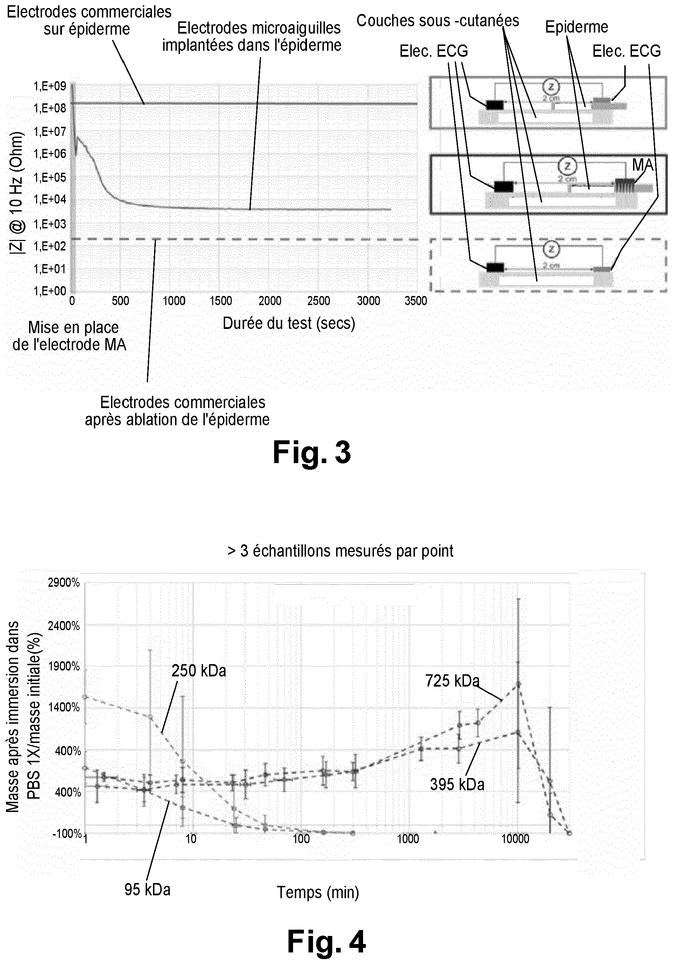

Les résultats sont décrits sur la Figure 3.

Les électrodes de référence ont un comportement similaire à celui qui pourrait être obtenu d'une mesure sur patient. L'impédance de mesure est élevée i.e. supérieure à 100 MΩ, si l'électrode est simplement posée sur l'épiderme tandis qu'elle est bien plus basse i.e. inférieure à 1kΩ) après abrasion de l'épiderme.

En comparaison, l'électrode micro-aiguilles selon l'invention montre une impédance élevée lors de son insertion, i.e. quand elle est sèche. Cette impédance diminue rapidement jusqu'à atteindre une valeur stable, comprise entre 0,1 et 1 kΩ, après environ 5 min.

En conclusion, l'impédance de mesure à l'aide d'une électrode micro-aiguille selon l'invention est améliorée de 4 ordres de grandeur par rapport à une référence ECG commerciale sans préparation préalable de la peau (i.e. abrasion de l'épiderme).

### III. Paramètres influençant les propriétés du dispositif selon l'invention.

### III.1. Polymère employé.

Par principe de fonctionnement, n'importe quel hydrogel possédant une forte capacité de gonflement en présence de liquide interstitiel peut être utilisé d'une manière similaire à celle décrite pour la CMC (carboxyméthylcellulose) réticulée par le CA (acide citrique). Il est supposé qu'un polymère ayant une capacité de gonflement importante permet une stabilisation plus rapide de la mesure qu'un polymère ayant une faible capacité de gonflement.

La masse molaire du polymère influe directement sur la capacité de gonflement du matériau, sa résistance mécanique et sa stabilité/dégradation. La Figure 4 et le Tableau 1 ci-dessous illustrent cette influence vis-à-vis du gonflement du matériau dans le cas de la CMC réticulée par 7,5% de sa masse d'acide citrique.

**Tableau 1 : Masse relative des gels CMC / acide citrique (7,5%) en fonction du temps d'immersion dans le PBS à 37°C.**

| Masse molaire CMC/ durée d'immersion dans PBS 1X (3 échantillons minimum) | 1-2 h | 7-8 h | 46-56 h | 300 h | 10 000 h | 30 000 h |
|---|---|---|---|---|---|---|
| 95 kDa | 680% ± 200 % | 210% ± 130 % | -5 2 % ± 56 % | -98 % ± 3 % | -100 % ± 0% | -100 % ± 0% |
| 250 kDa | 1540 % ± 330 % | 760% ± 780 % | -1 % ± 120 % | -94% ± 11% | -100 % ± 0% | -100 % ± 0% |
| 395 kDa | 573 % ± 52 % | 540% ± 150 % | 600% ± 140 % | 640% ± 110 % | 1110 % ± 850 % | -100 % ± 0% |
| 725 kDa | 460% ± 200 % | 480% ± 110 % | 540% ± 140 % | 640% ± 200 % | 1700 % ± 1100 % | -100 % ± 0 % |

D'après les résultats présentés à la Figure 4 et dans le Tableau 1, à taux de réticulation égal, un polymère de plus faible masse molaire se dissout en l'espace de quelques heures tandis que les polymères de plus haute masse molaire ont une stabilité de l'ordre de 2 semaines.

### III.2. Nature et densité de réticulation.

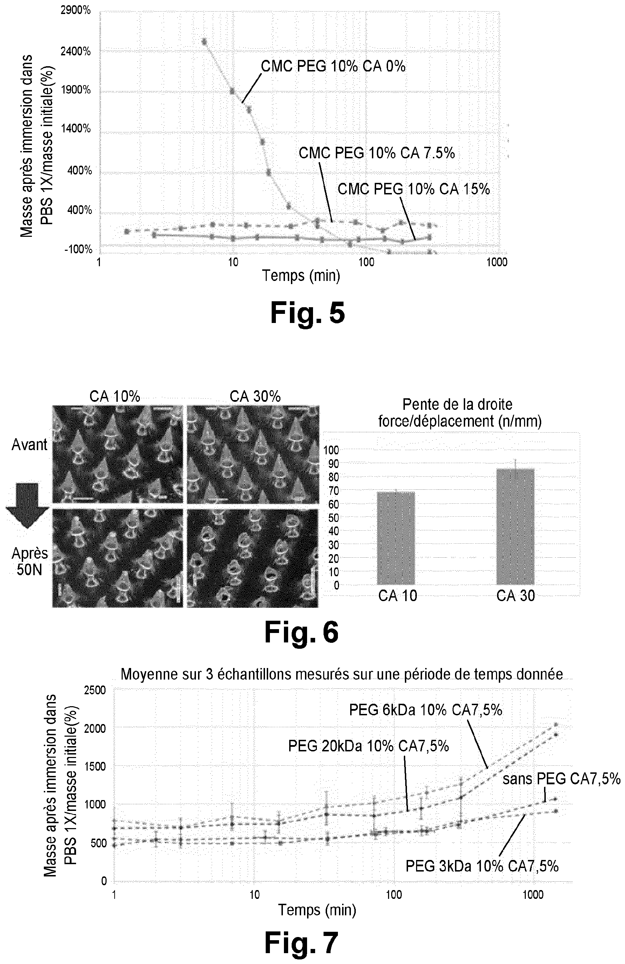

De plus, la capacité de gonflement des gels et leur stabilité est directement contrôlable par la quantité de réticulant introduite lors de leur mise en oeuvre, comme illustré à la Figure 5 et dans le Tableau 2 ci-dessous.

**Tableau 2 : Masse relative des gels CMC (725 kg/mol) / PEG (3,35 kg/mol) (10%) et à taux d'acide citrique variable en fonction du temps d'immersion dans le PBS à 37°C.**

| Quantité d'acide citrique relative à la masse de CMC / durée d'immersion dans PBS 1× (1 échantillon) | 6-7 h | 75-87 h | 300 h |
|---|---|---|---|
| 0% | 2523 % | 21% | -85 % |
| 7,5% | 263 % | 291 % | 250% |
| 15% | 116% | 77% | 107 % |

Ces résultats montrent qu'il est obligatoire de réticuler les gels CMC (i.e. les réticuler par l'acide citrique) afin d'éviter une dissolution rapide en milieu physiologique. La capacité de gonflement des gels ainsi formés est également limitée par la densité de réticulation.

La Figure 6 montre, dans sa partie gauche, des images de microscopies de micro-aiguilles sèches réalisées avec différents taux de réticulation avant et après une compression orthogonale de 50N. La partie droite de la Figure 6 montre la force nécessaire pour induire une déformation des aiguilles, i.e. leur rigidité.

On peut voir qu'en augmentant la quantité de réticulant acide citrique, le comportement mécanique du matériau évolue d'un comportement relativement ductile à un comportement rigide et cassant. Le contrôle des propriétés mécaniques permet d'optimiser la capacité de perforation des micro-aiguilles et de limiter leur probabilité de casse une fois insérées.

### III.3. Utilisation de plastifiants.

L'ajout de plastifiants dans un matériau polymère permet notamment de diminuer la densité de celui-ci. Ainsi, on peut modifier sa capacité de gonflement ainsi que sa stabilité.

**Tableau 3 : Masse relative des gels CMC (725 kg/mol) / CA (7,5%) de masse molaire variable en fonction du temps d'immersion dans le PBS à 37°C.**

| Masse molaire du PEG introduit dans la solution CMC/CA / durée d'immersion dans PBS 1X (3 échantillons mini) | 1 h | 10-15 h | 275-300 h |
|---|---|---|---|
| Sans PEG | 466 ± 30 % | 570 ± 80 % | 736 ± 45 % |
| PEG 3 kg/mol | 559 ± 56 % | 499 ± 21 % | 785 ± 97 % |
| PEG 6 kg/mol | 790 ± 170 % | 790 ± 130 % | 1261 ± 96 % |
| PEG 20 kg/mol | 688 ± 67 % | 750 ± 120 % | 1090 ± 240 % |

Dans les résultats présentés à la Figure 7 et dans le Tableau 3 ci-dessus, on peut voir que le PEG de basse masse molaire (3 kg/mol) n'apporte pas de bénéfices significatifs en termes de gonflement par rapport aux échantillons de contrôle sans PEG. On peut cependant voir une augmentation significative du taux de gonflement des gels, et ce, dès les premières minutes de l'immersion, des échantillons avec PEG de masse molaire 6 kg/mol et 20 kg/mol.

### Références bibliographiques

[1] Novel Hydrogel-Based Preparation-FreeEEG Electrode. Alba, Nicolas Alexander, et al., 2010, IEEE transactions on neural systems and rehabilitation engineering.
[2] Characterization of impédance properties of métal dry bioelectrodes with surface microstructure arrays. Zhou, Wei et al., 2017, Sensors and Actuators A : Physical.
[3] Design, fabrication and skin-electrode contact analysis of polymer microneedle-based ECG electrodes. O'Mahony, Conor, et al., 2016, Journal of Micromechanics and Microengineering.
[4] Recent advances of microneedles for biomedical applications: drug delivery and beyond. Yan, Jian, et al., 2019, Acta Pharmaceutica Sinica B.
[5] Biodegradable porous microneedles for an electric skin patch. Abe, Hiroya, et al. August 2021, Macromolecular Materials and Engineering, Vol. 306, P. 2100171.

## Revendications

1. Utilisation comme électrode pour un électrocardiogramme (ECG), pour un électro-encéphalogramme (EEG), pour un électromyogramme (EMG), pour un électro-oculogramme (EOG), pour un électrogastrogramme (EGG), pour une tomographie d'impédance électrique (EIT pour « Electrical impédance Tomography ») et pour une bio-impédance, d'un dispositif du type électrode comprenant
i) un patch ayant une première et une deuxième faces opposées,
ladite première face présentant des nano-structures et/ou des micro-structures,
ledit patch, lesdites nano-structures et lesdites micro-structures étant en un matériau comprenant au moins un polymère réticulé non-conducteur,
la surface externe des nano-structures et des micro-structures n'étant pas métallisée et
ii) un élément de reprise de contact électrique, appliqué sur ou intégré au niveau de ladite deuxième face.

2. Utilisation selon la revendication 1, **caractérisée en ce que** ledit au moins un polymère réticulé non-conducteur est au moins un biopolymère réticulé.

3. Utilisation selon la revendication 2, **caractérisée en ce que** ledit au moins un biopolymère réticulé est obtenu à partir d'un biopolymère choisi dans le groupe constitué par la méthylcellulose, l'éthylcellulose, la n-propylcellulose, l'hydroxyéthylcellulose, l'hydroxy-n-propylcellulose, l'hydroxy-n-butylcellulose, l'hydroxypropylméthylcellulose, l'éthylhydroxyéthylcellulose, la carboxyméthylcellulose, l'amidon de maïs, l'hydroxypropylamidon, le carboxyméthylamidon, le sulfate de dextrane, le phosphate de dextrane, du dextran acrylé, du dextran méthacrylé, le diéthylaminodextrane, l'héparine, le hyaluronane, la chondroïtine, le sulfate de chondroïtine, le sulfate d'héparane, l'acide polyglucuronique, l'acide polymanuronique, l'acide polygalacturonique et l'acide polyarabinique.

4. Utilisation selon l'une quelconque des revendications 1 à 3, **caractérisée en ce que** ledit matériau du patch comprend au moins un polymère réticulé non-conducteur et au moins un autre additif notamment choisi dans le groupe constitué par les plastifiants, les sels, les conservateurs et les colorants,
la masse totale dudit au moins un polymère réticulé non-conducteur étant, de préférence, supérieure ou égale à 75%, notamment supérieure ou égale à 80% et, en particulier, supérieure ou égale à 85% par rapport à la masse sèche totale du patch.

5. Utilisation selon l'une quelconque des revendications 1 à 4, **caractérisée en ce que** ledit élément de reprise de contact est choisi dans le groupe constitué par une électrode, une couche conductrice comme une couche métallique ; un ruban conducteur comme un ruban métallique; un adhésif conducteur; un fil conducteur comme un film métallique et une de leurs combinaisons.

6. Dispositif du type électrode comprenant :
(i) un patch ayant une première et une deuxième faces opposées, ladite première face présentant des nano-structures et/ou des micro-structures,
ledit patch, lesdites nano-structures et lesdites micro-structures étant en un matériau constitué d'un ou de plusieurs polymère(s) réticulé(s) non-conducteur(s) et éventuellement d'au moins un additif choisi dans le groupe constitué par les plastifiants, les sels, les conservateurs et les colorants, et
la surface externe des nano-structures et des micro-structures n'étant pas métallisée,
(ii) un élément de reprise de contact électrique, appliqué sur ou intégré au niveau de ladite deuxième face.

7. Procédé de préparation d'un dispositif selon la revendication 6 ou tel que défini à l'une quelconque des revendications 1 à 5, comprenant les étapes consistant à :
a) préparer une solution aqueuse contenant au moins un polymère réticulé non-conducteur ou au moins un de ses précurseurs et éventuellement au moins un additif ;
b) appliquer ladite solution préparée à l'étape a) sur un support ayant une forme et une géométrie, adaptées pour conférer, au patch obtenu après séchage de la solution aqueuse, une face nano-structurée et/ou micro-structurée ;
c) sécher ladite solution puis éventuellement soumettre ledit au moins un des précurseurs à des conditions permettant l'obtention dudit au moins un polymère réticulé non-conducteur moyennant quoi un patch est obtenu ;
ledit procédé comprenant, préalablement ou après cette étape c), une étape d'application sur ou d'intégration au niveau de la surface de la solution obtenue après l'étape b) ou de la face du patch à l'opposé de la face micro-structurée ou nano-structurée, d'un élément de reprise de contact électrique.

8. Procédé de préparation selon la revendication 7, **caractérisé en ce que** ledit au moins un précurseur dudit polymère réticulé non-conducteur est le polymère non réticulé non-conducteur et éventuellement au moins un agent de réticulation.

9. Procédé de préparation selon la revendication 8, **caractérisé en ce que** ledit agent de réticulation est choisi dans le groupe constitué par l'acide malonique, l'acide malique, l'acide maléique, l'acide succinique, l'acide glutarique, l'acide adipique, l'acide citrique, l'acide isocitrique, l'acide aconitique, l'acide propane-1,2,3-tricarboxylique et l'acide 2,3,3',4'-biphényltétracarboxylique.

10. Procédé de préparation selon la revendication 8, **caractérisé en ce que** ledit agent de réticulation est un photo-initiateur.

11. Procédé selon l'une quelconque des revendications 7 à 10, **caractérisé en ce que** ladite étape a) consiste à :
- ajouter, à un solvant aqueux comme de l'eau ou de l'eau distillée, un ou des polymère(s) non réticulé(s) non-conducteur(s) et éventuellement un ou plusieurs additif(s) puis mélanger l'ensemble, sous agitation notamment générée par une roue tournante, pendant une durée et à une température permettant d'obtenir un mélange homogène ;
- ajouter à la solution aqueuse contenant un ou des polymère(s) non réticulé(s) non-conducteur(s) et éventuellement un ou plusieurs additif(s) ainsi obtenue, au moins un agent de réticulation puis mélanger l'ensemble, sous agitation notamment générée par un vortex ou un barreau aimanté, pendant une durée et à une température permettant d'obtenir un mélange homogène.
11. Système porté pour le suivi en ambulatoire d'un patient, comprenant un dispositif selon la revendication 6 ou tel que défini à l'une quelconque des revendications 1 à 5.
